# EUROPEAN PATENT SPECIFICATION

(11) **EP 3 772 322 B1**
(45) Date of publication and mention of the grant of the patent: **10.01.2024**
(21) Application number: 20189948.1
(22) Date of filing: 06.08.2020
(51) Int. Cl.: A61B 1/00

(54) **PROXIMAL LOCK ADAPTER FOR A SCOPE**
ADAPTER ZUR PROXIMALEN VERRIEGELUNG FÜR EIN ENDOSKOP
ADAPTATEUR DE VERROUILLAGE PROXIMAL POUR UN OSCILLOSCOPE

(30) Priority: 06.08.2019 DE 102019121269
(43) Date of publication of application: 10.02.2021
(73) Proprietor: Delmont Imaging SAS, 13600 La Ciotat (FR)
(72) Inventor: Montillot, Pierre, 13600 La Ciotat (FR); Buck, Bernd, 72488 Sigmaringen (DE); Brikow, Eugen, 88630 Pfullendorf (DE); Schulz, Dieter, 78600 Kolbingen (DE)
(74) Representative: Bausch, Thomas

(56) References cited:
- WO-A1-2011/060192
- WO-A1-2013/110073
- US-A- 6 117 070
- US-B1- 6 196 967

## Description

The present invention relates to hysteroscopic and resectoscopic systems. Typically such systems comprise different traditional resectoscopy surgical capabilities, by combining and integrating them into an endoscope that also enables gynecologic mechanical and electro-surgical instruments to be used. Usually it is connected with a light source and an irrigation system. It can be operated by one hand and is typically equipped with an optical unit with an offset eyepiece, a rigid operating channel which enables the insertion of mechanical instruments and electrodes that are both monopolar and bipolar. It continually guarantees a continuous irrigation flow and consequently a clear operating field.

In a typical application a hysteroscope is inserted into the cavum uteri which has to be inflated for inspection or for any surgical procedures. For inflation a special liquid or a gas has to be conveyed through the hysteroscope sheath and, after the inflation has been performed, the pressure in the cavum uteri has to be kept on a defined level.

Typical surgical procedures include endocavitary pathologies such as uterine sessile or pedunculated polyps which are resected with monopolar or bipolar electrodes. In these cases the hysteroscope is operated as an resectoscope.

Resectoscopes do have similar equipment and are used for various medical operations. The instruments can be combined or used as single application. In the following those various types of endoscopes are summarized as scopes.

Hysteroscopes are well known state of the art. Early CH 295527 relates to an endoscope and in particular to a rectoscope, hysteroscope, bronchoscope and similar medical instruments, with a tube to be inserted into the body opening and a lighting device for illuminating the parts of the body cavity surrounding the end of the tube.

DE OS 2 318 860 describes a hysteroscope, suitable for use with a cervical adaptor comprising a barrel for yielding a viewing system and having an inlet passage for feeding insufflation gas into the barrel, for spreading apart the walls of the uterus, and means for holding the uterine walls in their spread apart condition when the introduction of insufflation gas is discontinued. This spreading devices include a plurality of spreader arms which are biased away from each other. They are carried on an annular member which can slide axially in the barrel between a retracted position in which the arms are displaced towards each other by the distal end of the barrel and a spread-out position in which the arms, having been freed from the restraint of the distal end of the barrel and are shifted away from each other, hence contacting the uterine walls. Furthermore an inlet for a probe may be provided. The spreader arms may have the form of closed loops.

DE 20 02 829 C2 relates to a hysteroscope of the type comprising an outer envelope forming a cannula, means for insufflation of a fluid, particularly a gaseous fluid, such as for example carbon dioxide, inside the uterine cavity distending the walls thereof, and an inner envelope forming an endoscope tube, of smaller diameter than the outer envelope and adapted to slide therein, this inner envelope containing light transmission means for conducting light to the distal end of said inner envelope and comprising on the one hand means for placing mechanically the hysteroscope in operating position, substantially fluid-tight at the level of the cervix of the uterus and on the other hand a portable device for supplying fast said fluid, with said hysteroscope.

US 5,320,091 A shows a continuous flow hysteroscope. This hysteroscope includes an outer sheath having an outer surface, an inner surface and a hollowed-out central area extending along a central axis which extends there through. The outer sheath comprises a proximal section and a distal section. The distal end includes a plurality of openings communicating between the outer surface and inner surface. The openings have a predetermined shape and pass fluid exterior to the distal section therethrough and into the hollowed-out central area. The proximal section of the outer sheath includes an outlet and an inlet. An inner member is positioned within the hollowed central area and includes a first channel which is adapted to receive a telescope. A second channel, which is substantially parallel to the first channel is utilized for passing a working tool. The first and second channel are operatively coupled to an inlet for passing fluid there through and out of the distal end of the outer sheath. The fluid passageway cooperates with the plurality of openings to pass fluid through the opening from the outer surfaces to the inner surfaces, through the fluid passageway and through the outlet of the outer sheath. The inner member includes an obturator closure device to isolate inflow and outflow distally, to prevent cross flow and to reduce patient trauma at time of insertion of the hysteroscope.

EP 1 325 704 B1 describes a hysteroscope with a shank exchange system. It has an optics housing provided at a proximal end, with an outer shank releasably connected to the optics housing, and with an inner shank which is arranged inside and parallel to the outer shank and is releasably connected to the optics housing. A corresponding endoscope set and a set of endoscope shanks are also provided.

WO 2011/150111 A1 shows an endoscope with a hollow outer sheath configured for insertion into a patient's body. A hollow inner sheath is receivable within the outer sheath, and is configured to slideably receive a first internal instrument. A working element can be configured to rotatably support at least the first internal instrument and a second internal instrument such that at least the first internal instrument and the second internal instrument are rotatable around a common rotation axis being substantially coextensive with a longitudinal axis of the outer sheath. The working element can have at least one guide rail extending longitudinally of the axis of rotation and an actuator block can be slideably mountable to the at least one guide rail and so securable to at least one of the instruments as to be able to push the at least one of the internal instruments longitudinally of the outer sheath.

WO 2011/060192 A1 describes a surgical access device comprising a working channel providing a first lumen having an axial opening for insertion of a surgical instrument and having an outflow channel, the first lumen having a fluid inlet for allowing fluid flow through the working channel and into the body, wherein fluid flows out of the body through the outflow channel of the surgical instrument. The surgical access device can further comprise a seal apparatus coupled to the working channel and configured to receive the surgical instrument, the seal apparatus comprising a first seal portion and a second seal portion, the first seal portion configured to close the fluid path into the seal apparatus, and the second seal portion configured to form a seal with the surgical instrument.

Despite the fact that this endoscopic technology is known for a long time and its use is very common, some serious problems concerning the irrigation could yet not be solved sufficiently. On the one hand the valves of the hysteroscope connecting it with the inlet flow and with the outlet flow have to be very tight but easy going at the same time and the handling should be very easy despite the fact that the hoses filled with pressurized fluid are heavy, bulky, unwieldy and sometimes even inflexible. Those hoses therefore apply unwanted force and momentum to the connections of the hysteroscope and impede the operating person, especially when moving the position.

During usage of the hysteroscope the operating person should be able to change instruments without releasing the pressure of the irrigation and without losing the inflating effect of the uterus. The operator has to resect, grasp, extract respectively remove material while irrigation is still running continuously. The connection of the irrigation system should allow to turn the hysteroscope about a longitudinal axis by one hand while the other hand of the surgeon is coping with the devices and exchanging these instruments for different operation steps. During the surgical procedure the surgeon needs his view of the operating field respective for the visualization system of the endoscope and no devices of the irrigation system should distract his attention.

The most advanced technology to solve that functionality is the endoscopic system of HEOS, Hysteroscopy Endo-operative System, as described by Acc. Dr. R. Ricciardi, "HEOS - In respect of women, Sopro Comeg 2017". It solves many of these requirements by a hysteroscopic system, comprising a straight forward rigid hysteroscope, having a main body with parallel eyepiece, a light source adapter for connecting a light source, a tube having a distal end with a distal window, this tube having a fiber optic light transmission incorporated and a rod lenses optical system, and being equipped with at least one working channel and an irrigation system with separate channels for inflowing and outflowing fluid, as well as a hysteroscopy sheath connecting the tube and the main body.

But cleaning and sterilization of that instrument is expensive and difficult. The instrument is equipped with a multitude of valves. All valves do have small dead spaces and tiny dead volumes where infectious particles can accumulate during procedure. All bearings which allow axial turning of the scope are potential subject of contamination and all sealings could leak. The smaller the diameters of the whole hysteroscope are, the better for the patient, but the worse for the solidity and the tightness of the instrument. Deformation in the micrometer scale as a result of mechanical stress leads to small leakings. Unfortunately these little deformations disappear during sterilization, as little openings are closing with mechanical stress releasing, and captured microbes can survive. This problem is the reason why many parts of that instrument are designed as spare parts for one-time use and which have to be replaced for every new use, after having been removed and disposed of before sterilization.

The task of the invention therefore is, to improve this hysteroscopic system in a way that these disadvantages are avoided. This solution should work with rigid endoscopes as well as with flexible endoscopes.

The solution is a proximal lock adapter for hysteroscopic or resectoscopic systems, those systems comprising
- a main body with a parallel eyepiece, a light source adapter for connecting a light source,
- a tube having a distal end with a distal window,
- that tube having a fiber optic light transmission and an optical system with rod lenses or fibres incorporated, and equipped with at least one working channel,
- that tube fixed with the main body,
- and an irrigation system with separate channels for inflowing and outflowing fluid,
- means for irrigating fluid at the distal end of the tube,
- means for insertion of resecting instruments from the proximal end,
- a screw thread at the proximal end of the main body,
where the lock adapter is provided at the proximal end of the working channel, this lock adapter comprising
- a working channel,
- an instrument connection end piece with an external thread at the distal side,
- a screw sleeve with an internal thread and an external thread,
- a stopcock and stopcock ventilation,
- a double seal of silicone,
where the screw sleeve is attached to the screw thread of the main body and to the external thread of the instrument connection end piece.

This means that instruments can be inserted into the working channel while the irrigation is still running and the cavity remains pressurised and inflated without causing leakage at the proximal side, even when changing instruments.

Besides, even the instruments used for surgical operation can be rotated in the working channel independently from the position of the main body or from the irrigation system. This is achieved by a double seal of silicone with predetermined breaking lines at the proximal end of the working channel. If the instrument is inserted from the proximal opening of the working channel it breaks through the predetermined breaking lines made of silicon, and when it is pulled out these breaking lines close by adhesion until the next instrument is inserted. No leakage occurs.

Before the next patient is treated or before the next hysteroscopy or resectoscopy takes place, these double seal is replaced by a new one, the used double seal is disposed of. And as no part of the working channel and no thread for assembling comes into contact with infectious material no sterilisation problems occur and in cases when larger particles have to be pulled by instruments through the working channel, sterilisation is easy after disassembling the proximal lock adapter.

Additionally, even the proximal lock adapter end piece can be rotated in that way that the stopcock and the stopcock ventilation can be adjusted in a position comfortable to the surgeon. For that purpose the end piece is designed to comprise a screw sleeve, a stopcock and stopcock ventilation which are assembled in a way that the screw sleeve is attached to the screw thread of the main body adapter which is connected to the main body.

Optionally an additional working channel can be provided similar to the state of the art at the main body adapter. This can be equipped with a stopcock and it can be used to insert a flexible instrument into the working channel or to provide additional irrigation if necessary.

The drawings briefly described below show the invention in more detail without limiting it to the examples shown:
- Fig. 1:: A sketch of the scope from the left side,
- Fig. 2:: A perspective sketch of the scope with irrigation system,
- Fig. 3:: A sectional drawing of a first alternative of the proximal lock adapter
- Fig. 4:: A sectional assembly drawing of a second alternative of the proximal lock adapter,
- Fig. 5:: A sectional drawing of the second alternative of the proximal lock adapter (mounted)
- Fig. 6a:: A sectional assembly drawing of the silicone sealings with dome sealing,
- Fig. 6b:: A sketch of the first sealing from proximal,
- Fig. 6c:: A sketch of the second sealing with dome sealing from distal,
- Fig. 6d:: A sectional assembly drawing of the silicone sealings with cone sealing,
- Fig. 6e:: A sketch of the first sealing from proximal,
- Fig. 6f:: A sketch of the second sealing with cone sealing from distal.

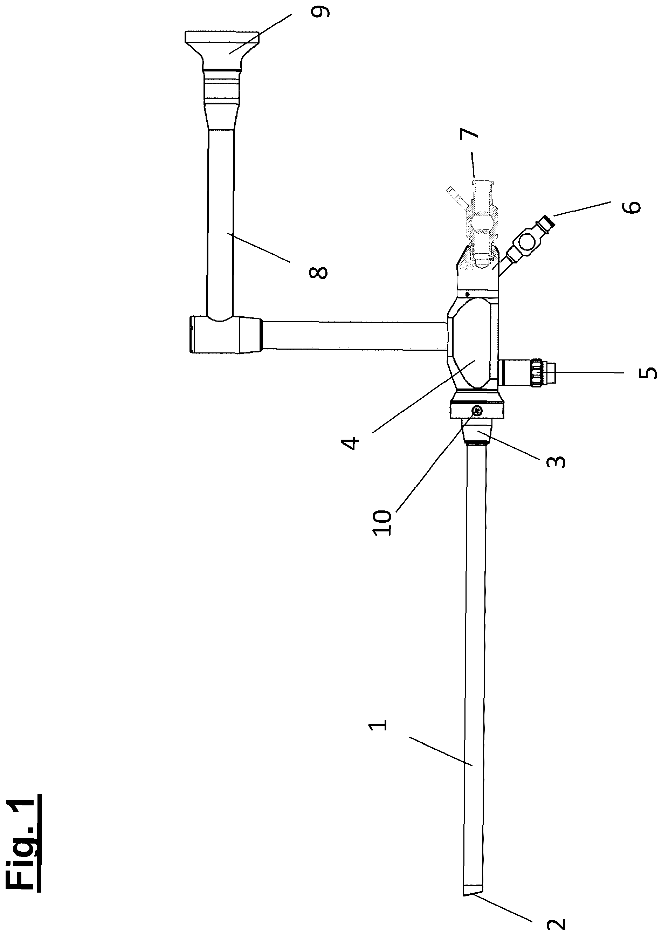

Fig. 1 shows a sketch of the scope as it is seen from the left hand side of the operator. The outer tube 1 has a distal opening 2 with a distal window, an illumination means, optical means and at least one working channel for instruments and irrigation. The outer tube 1 is connected with the main body 4, the main body 4 is also connected to the quick lock adapter 3 and to the connection for illumination fibre 5. The scope is equipped with a proximal lock adapter 7 and an additional working channel 6, which is optional. An optic rod 8 lense is arranged parallel to the main working channel to allow the operator to use the instruments while looking into the eyepiece 9 and to observe the surgical work at the same time.

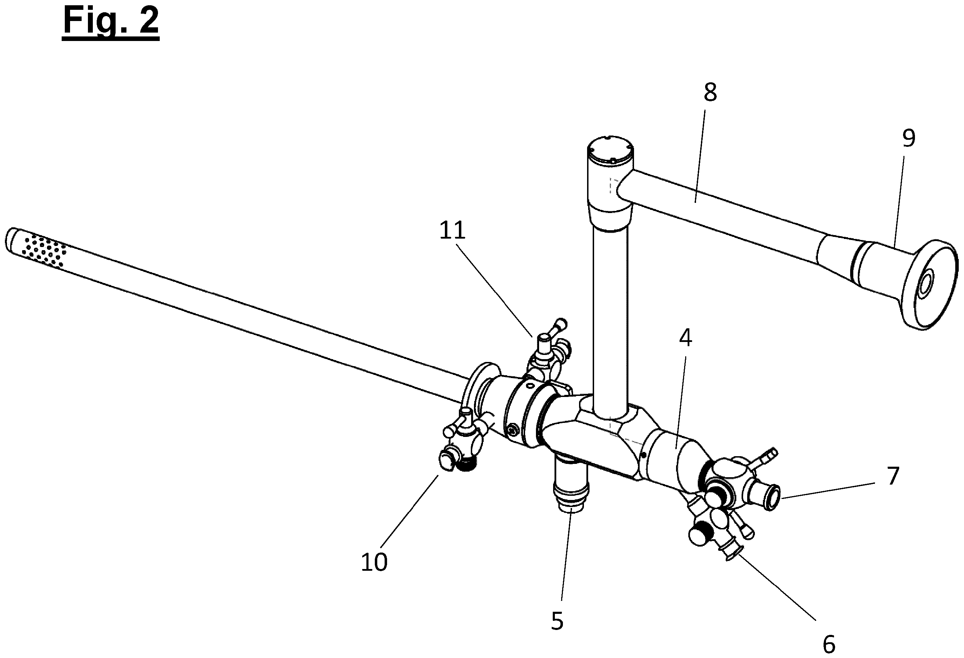

Fig. 1 does not show the irrigation system nor the surgical instruments. But for connecting the irrigations system the quick lock fixation 10 is shown. Fig. 2 shows the scope after mounting the irrigation system with the outer tube.

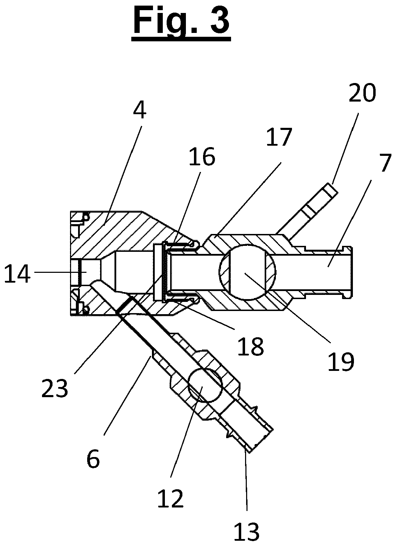

Figs. 3 to 5 show different alternatives for the connection of instruments.

Fig. 3 shows a proximal lock adapter connection for instruments which his equipped with a stopcock 19, so the working channel 14 can be closed. After closing that valve, a ventilation 20 balances pressure. Additionally an adapter with screw sleeve 18 is used for easy disassembling the instrument connection end piece 17.

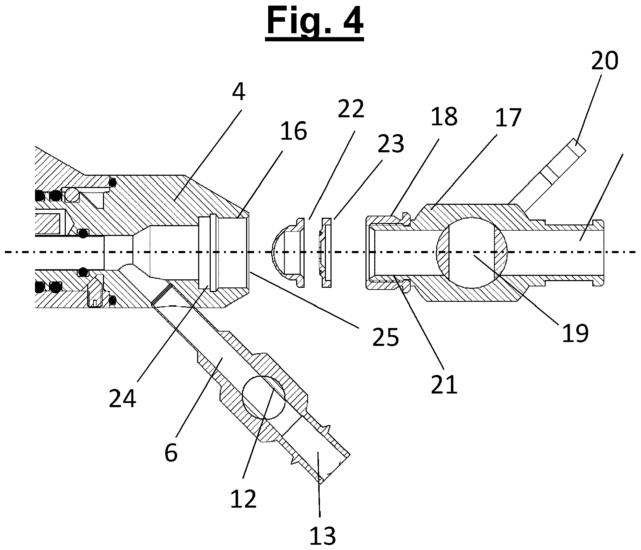

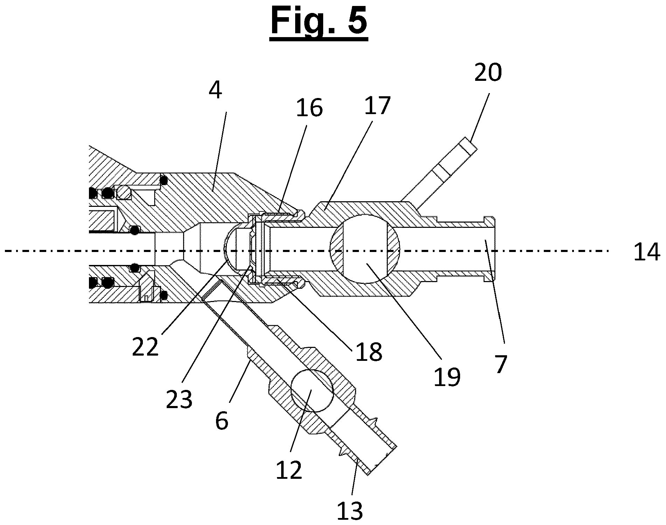

Fig. 4 shows an advanced proximal lock adapter as an assembly drawing of the silicone sealings. Two silicone sealings, an inner silicone valve 22 and an outer silicone sealing 23, are used. For the inner silicone valve 22 the main body adapter 15 is equipped with an overhang space 24 which fits exactly to the outer diameter of the inner silicone valve. The outer silicone seal fits exactly to the surface of the inner silicone valve, the outer silicone valve 23 is fixed by the external thread of the instrument connection end piece 17. Fig. 5 shows the assembled object of Fig. 4.

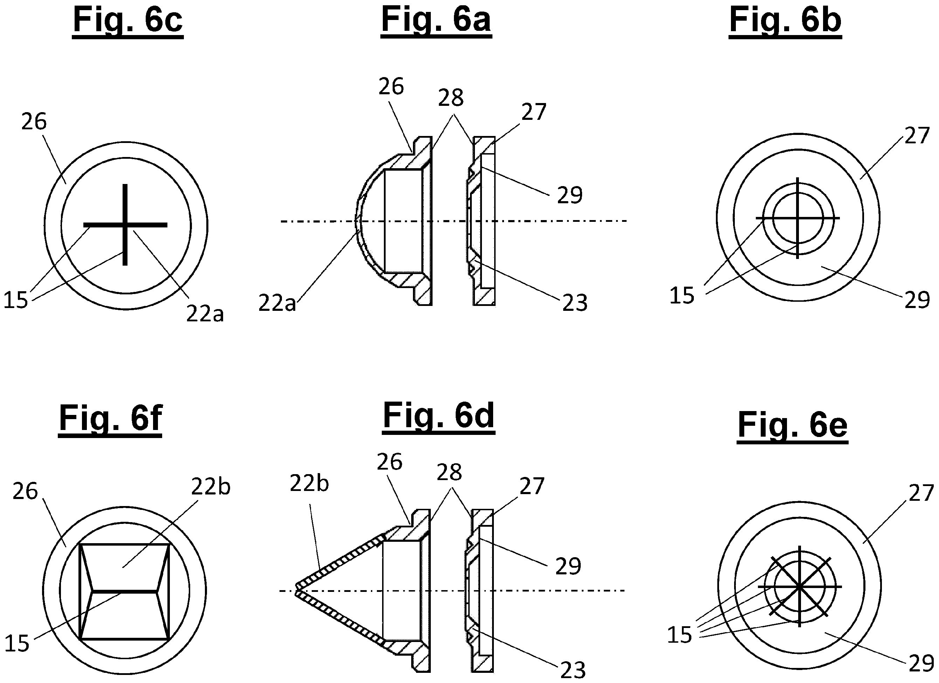

Figs. 6a to 6f show to alternatives of the inner, distal silicon valve 22 (22a and 22b) and the outer, proximal silicone sealing 23 in more detail.

Fig. 6a shows the inner silicone dome valve 22a, the dome showing to the main body direction. It has an annular edge 26 which fits to the overhang space 24 shown in Fig. 4. On the opposite side the sealing surface 28 fits to the silicone seal 27 of the outer silicone sealing 23. While the silicone seal 27 and the sealing surface 28 are hold by the screw sleeve 18, the smaller sealing surface 29 is hold by the instrument connection end piece 17.

Fig. 6b relates to outer silicone sealing 23 and has two predetermined breaking lines 15, which are arranged crosswise. If an instrument is inserted into the working channel 14 these breaking lines are ruptured and open the path for the instrument. If another instrument should be used, the first instrument can be replaced without replacing the outer silicone sealing. All the inserted instruments can be rotated and axially moved.

Fig. 6c relates to the inner silicone dome valve 22a which has another two predetermined breaking lines 15, which are arranged crosswise so that they each open a quarter of a half sphere when the instrument is put through. If the instrument is drawn back and out, the inner pressure of the working channel 14 makes the dome openings close and no irrigation fluid can leak.

Fig. 6d relates to the inner silicone check valve 22b, the cone form as being an alternative to the dome form. Fig. 6e relates to outer silicone sealing 23 and has four predetermined breaking lines 15, which are arranged cake-like, of course any other number of breaking lines can be used as well. Fig. 6f shows the silicone check valve 22b. The form of the check valve can be adapted to the instruments which are to be used.

The examples shown can be used with rigid endoscopes as well as with flexible endoscopes and can be used even alternately with both types of endoscopes when the connections of the main bodies are the same and both fit to the same proximal lock adapter.

Another advantage is that none of the screw threads can come into contact with materials in the working channel because the sealing surfaces 28 and 29 keep any contamination away from the screw threads. This facilitates cleaning and sterilization after usage.

### Reference list

- 1: Outer tube
- 2: Distal opening
- 3: Quick lock adapter
- 4: Main body
- 5: Connection for illumination fibre
- 6: Additional working channel
- 7: Proximal lock adapter
- 8: Fibre optic rod
- 9: Eyepiece
- 10: Quick lock fixation
- 11: Quick lock push button
- 12: Additional working channel stopcock
- 13: Opening of additional channel
- 14: Working channel
- 15: Predetermined breaking line
- 16: Screw thread
- 17: Proximal lock adapter end piece
- 18: Screw sleeve
- 19: Stopcock
- 20: Stopcock ventilation
- 21: External thread
- 22: Inner silicone valve
- 22a: Inner silicone dome valve
- 22b: Inner silicone check valve
- 23: Outer silicone sealing
- 24: Overhang space
- 25: Internal thread
- 26: Edge
- 27: Silicone seal
- 28: Sealing surface
- 29: Sealing surface

## Claims

1. Lock adapter for hysteroscopic or resectoscopic systems, those systems comprising
• a main body (4) with a parallel eyepiece (9), a light source adapter (5) for connecting a light source,
• a tube (1) having a distal end (2) with a distal window,
• that tube (1) having a fiber optic light transmission and an optical system with rod lenses or fibres incorporated, and equipped with at least one working channel,
• that tube (1) fixed with the main body (4),
• and an irrigation system with separate channels for inflowing and outflowing fluid,
• means for irrigating fluid at the distal end (2) of the tube (1),
• means for insertion of resecting instruments from a proximal end of the working channel of the tube,
• a screw thread (16) at the proximal end of the main body,
wherein
the lock adapter, in use, can be provided at the proximal end of the working channel of the tube, the lock adapter (7) comprising
• a working channel (14),
• an instrument connection end piece (17) with an external thread (21) at the distal side,
• a screw sleeve (18) with an internal thread and an external thread,
• a stopcock (19) and stopcock ventilation (20),
• a double seal of silicone (22, 23),
where the screw sleeve (18) is attachable to the screw thread (16) of the main body (4) of the hysteroscopic or resectoscopic system and to the external thread (21) of the instrument connection end piece (17).

2. Lock adapter according to claim 1, **characterised in that** the double seal of silicone (22, 23) has predetermined breaking lines (25).

3. Lock adapter according to any of claims 1 or 2, **characterised in that** both seals forming the double seal (22, 23) have an outer ring part which fits exactly into the screw thread (16) of the main body and an inner part which has predetermined breaking lines (15).

4. Lock adapter according to claim 1, **characterised in that** the seal of silicone (23) at the proximal side is structured with a flat cross-section.

5. Lock adapter according to claim 1, **characterised in that** the seal of silicone (22) at the distal side is structured with a dome cross-section, the dome directed to distal direction.

6. Lock adapter according to claim 1, **characterised in that** the seal of silicone (22) at the distal side is structured with a cone cross-section, the cone directed to distal direction.

## Patentansprüche

1. Schließ-Adapter für hysteroskopische oder resektoskopische Systeme, wobei diese Systeme umfassen:
• einen Hauptkörper (4) mit einem parallelen Okular, (9), einen Lichtquellenadapter (5) zum Verbinden einer Lichtquelle,
• ein Rohr (1) aufweisend ein distales Ende (2) mit einem distalen Fenster,
• das Rohr (1) aufweisend eine faseroptische Lichttransmission und ein optisches System mit eingebetteten Stablinsen oder Fasern, und mit mindestens einem Arbeitskanal ausgestattet,
• das Rohr (1) mit dem Hauptkörper (4) fixiert ist,
• und ein Spülsystem mit separaten Kanälen für einströmendes und ausströmendes Fluid,
• Mittel zum Spülen von Fluid am distalen Ende (2) des Rohres (1),
• Mittel zum Einführen von Resektionsinstrumenten von einem proximalen Ende des Arbeitskanals des Rohres,
• ein Schraubgewinde (16) am proximalen Ende des Hauptkörpers,
der Schließ-Adapter, im Gebrauch, am proximalen Ende des Arbeitskanals eingesetzt werden kann, wobei dieser Schließ-Adapter umfasst:
• einen Arbeitskanal (14),
• ein Instrumentenverbindungsendstück (17) mit einem Aussengewinde (21) an der distalen Seite,
• eine Schraubhülse (18) mit einem Innengewinde und einem Aussengewinde,
• einen Absperrhahn (19) und eine Absperrhahnentlüftung (20),
• eine Doppeldichtung aus Silikon (22,23),
wobei die Schraubhülse (18) an dem Schraubgewinde (16) des Grundkörpers (4) und an dem Aussengewinde(21) des Instrumentenanschlussendstücks (17) befestigbar ist.

2. Schließ-Adapter nach Anspruch 1, **dadurch gekennzeichnet, dass** die Doppeldichtung aus Silikon (22,23) Sollbruchlinien (25) aufweist.

3. Schließ-Adapter nach einem der Ansprüche 1 oder 2, **dadurch gekennzeichnet, dass** beide Dichtungen, die die Doppeldichtung (22, 23) bilden, ein äusseres Ringteil aufweisen, das genau in das Schraubgewinde (16) des Hauptkörpers passt, und ein Innenteil, das vorbestimmte Sollbruchlinien (25) aufweist.

4. Schließ-Adapter nach Anspruch 1, **dadurch gekennzeichnet, dass** die Dichtung aus Silikon (23) an der proximalen Seite mit einem flachen Querschnitt strukturiert ist.

5. Schließ-Adapter nach Anspruch 1, **dadurch gekennzeichnet, dass** die Dichtung aus Silikon (23) an der distalen Seite mit einem Domquerschnitt strukturiert ist, wobei der Dom in distale Richtung gerichtet ist.

6. Schließ-Adapter nach Anspruch 1, **dadurch gekennzeichnet, dass** die Dichtung aus Silikon (22) an der distalen Seite mit einem Konusquerschnitt strukturiert ist, wobei der Konus in distale Richtung gerichtet ist.

## Revendications

1. Adaptateur de verrouillage pour systèmes hystéroscopiques ou résectoscopiques, ces systèmes comprenant
• un corps principal (4) avec un oculaire parallèle (9), un adaptateur de source de lumière (5) pour connecter une source de lumière,
• un tube(1) ayant une extrémité distale (2) avec une fenêtre distale,
• ce tube (1) ayant une transmission de lumière à fibre optique et un système optique à lentilles ou fibres de tige incorporées, et équipé d'au moins un canal de travail,
• ce tube (1) fixé au corps principal (4),
• et un système d'irrigation avec des canaux séparés pour l'entrée et la sortie de fluide,
• des moyens d'irrigation de fluide à l'extrémité distale (2) du tube (1),
• des moyens d'insertion d'instruments de résection à partir de l'extrémité proximale du tube (1) à l'extrémité distale (2) du tube (1),
• un filetage (16) à l'extrémité proximale du corps principal,
un adaptateur de verrouillage utilisé est prévu au niveau de l'extrémité proximale du canal de travail, cet adaptateur de verrouillage (7) comprenant
• un canal de travail (14),
• une pièce d'extrémité de connexion d'instrument (17) avec un filetage externe (21) au niveau du côté distal,
• un manchon de vis (18) avec un filetage interne et un filetage externe,
• un robinet d'arrêt (19) et une ventilation d'arrêt (20),
• un double joint d'étanchéité en silicone (22, 23),
où les manchons de vis (18) sont fixés au filetage de vis (16) du corps principal (4) et au filetage externe (21) de l'embout de raccordement d'instrument (17).

2. Adaptateur de verrouillage selon la revendication 1, **caractérisé en ce que** le double joint de silicone (22, 23) a des lignes de rupture prédéterminées (25).

3. Adaptateur de verrouillage selon l'une quelconque des revendications 1 ou 2, **caractérisé en ce que** les deux joints formant le double joint (22, 23) ont une partie annulaire externe qui s'ajuste exactement dans le filetage (16) du corps principal et une partie interne qui a lignes de rupture prédéterminées (25).

4. Adaptateur de verrouillage selon la revendication 1, **caractérisé en ce que** le joint de silicone (23) au niveau du côté proximal est structuré avec une section transversale plate.

5. Adaptateur de verrouillage selon la revendication 1, **caractérisé en ce que** le joint de silicone (22) au niveau du côté distal est structuré avec une section transversale de dôme, le dôme étant dirigé vers la direction distale.

6. Adaptateur de verrouillage selon la revendication 1, **caractérisé en ce que** le joint de silicone (22) au niveau du côté distal est structuré avec une section transversale conique, le cône étant dirigé vers la direction distale.
